# EUROPEAN PATENT SPECIFICATION

(11) **EP 0 728 492 B1**
(45) Date of publication and mention of the grant of the patent: **03.01.2001**
(21) Application number: 96102778.6
(22) Date of filing: 24.02.1996
(51) Int. Cl.: A61M 5/315

(54) **Dual chamber internal by-pass syringe assembly**
Zweikammerspritze mit internem Bypass
Seringue à double chambre avec court-circuit interne

(30) Priority: 27.02.1995 US 394712; 14.02.1996 US 599906
(43) Date of publication of application: 28.08.1996
(73) Proprietor: Schott Parenta Systems, Inc., Yonkers, NY 10701 (US)
(72) Inventor: Michaels, Thomas M., Upper Saddle River, NJ 07458 (US)
(74) Representative: Patentanwälte Westphal, Mussgnug & Partner

(56) References cited:
- EP-A- 0 415 867
- DE-A- 1 566 644
- GB-A- 2 252 951
- US-A- 4 613 326

## Description

### FIELD OF THE INVENTION

The present invention relates to improvements in syringe assemblies. More particularly the invention relates to improvements in so called single barrel, two compartment syringes wherein two components of a medicament, such as two liquids or a solid and a liquid can be packaged in the syringe each in its own sealed environment and wherein the components can be mixed when desired so that they can be dispensed.

### BACKGROUND OF THE INVENTION

The use of syringes to administer medicaments to patients has long been recognized as an effective method of treatment. In some instances, where the medicaments are in powder form and need to be reconstituted by the addition of distilled water or other liquids, administration of the medicament involves a number of steps and the use of multiple syringes. For example, when the components of a medicament are to be mixed prior to administration, in some instances the procedure involves (1) insertion of a syringe into a first container having the first component, (2) withdrawing the first component into the syringe, (3) withdrawing the syringe from the first container, (4) insertion of the syringe into a second container having a second component, (5) discharge of the first medicament into the second container, (6) mixing the two components within the second container, (7) withdrawing the mixed medicament, and (8) administering the medicament to the patient. It is noted that it would be a great advantage to the art if many of these steps can be combined or avoided all together.

Methods have been proposed to avoid the above described difficulties through use of dual chamber syringes which employ an external by-pass. Several of these are manufactured for use in packaged drugs. The drugs typically are liquids contained in each chamber of the dual chamber syringe, whereby the two liquids are mixed prior to administration. These dual chamber syringes have been used for packaging lyophilized drugs that are sublimated in the syringe. The dual chamber syringe's use of an external by-pass, however, can cause mixing and surging problems. Some drugs remain in the external by-pass and can contaminate exposed areas of the syringe. Also, if the medicament is toxic or corrosive, the preparer and user of the syringe is put at an undesirable risk.

Another known design vents to permit passive mixing of a liquid and a solid in one chamber of the syringe. This design employs a central plunger having a burstable diaphragm which is designed to create turbulence upon breaking loose of the diaphragm and activation of the stopper to enhance mixing of the materials in the tube chambers. A problem with this system is the problem of particulates created when the diaphragm bursts which may be a hazard to the patient if injected with the medicament.

Another internal stopper design includes dual compartment vials held in place by a constriction in the body of the vial. In this instance, the stopper serves as a barrier and is dislodged after activation to permit the two materials in the dual compartments to co-mingle and combine.

Another known syringe device is a combination syringe and vial. The stopper closing the vial is held in place by a constriction and is vented to permit introduction of a liquid into the vial. The stopper serves as a plunger when the contents of the syringe are expelled.

Other attempts to achieve an adequate design have used modifications of the plunger rod in conjunction with a syringe. The syringe is activated by threading the plunger rod toward the proximal end which breaks the by-pass stopper loose and moves it slowly past the external by-pass. The plunger rod only moves freely as in a traditional syringe after the last male thread on the plunger rod passes the last female thread in the finger grip.

Document DE-A-1566644 upon which the preamble of claim 1 is based, describes a two compartment syringe comprising an elongated hollow tubular barrel having a discharge opening at one end, a radially inwardly directed circumferentially extending rib in the inner peripheral surface of said barrel and an inner cylinder comprising a valve extending axially through the center of the inner cylinder. The valve allows fluid communication between the chambers when by actuating a plunger the pressure in one of the chambers exceeds the pressure in the other chamber. A circumferentially extending groove of the inner cylinder in correspondence with said rib serves to prevent the inner cylinder from being displaced axially to the rib while fluid flows through the valve.

Document EP-A-0415867 describes a syringe for unique use. The syringe comprises an elongated hollow tubular barrel having a discharge opening at one end, a radially inwardly directed circumferentially extending rib in the inner peripheral surface of said barrel and a moving body movable in the barrel. The moving body comprises a flexible circular edge which allows the body to pass the rib in the direction of the discharge opening when a plunger is actuated. After having passed the rib, the flexible edge prevents the body to pass the rib in the opposite direction. If the plunger is then moved to aspire liquid the body and the rib form a sealing to prevent the reuse of the syringe.

Although various syringes are known, it would be a great advance in the art if a simple, effective dual chamber syringe could be provided which would overcome the deficiencies of those designs presently known in the industry.

### SUMMARY OF THE INVENTION

With the above in mind, it is an object of the present invention to provide an improved single barrel, two compartment syringe for storing and dispensing plural component medicaments. This is achieved by a syringe assembly including the features of claim 1. The syringe assembly includes an elongated hollow syringe barrel. The forward end of the syringe barrel mounts a conventional double ended needle and hub assembly and is normally in an unarmed position so that the discharge opening is sealed. The opposite end of the syringe is open to receive a plunger and plunger rod moveable axially in the syringe barrel to displace the contents of the syringe when it is desired in the manner described hereafter. The syringe barrel is divided into a medicament chamber and diluent chamber by a by-pass stopper which has a circumferentially extending sealing surface which normally engages an internal rib in the syringe barrel to hermetically seal the chambers from one another an a plurality of axially extending, radially outwardly projecting ribs on the proximal side or both sides of the sealing surface which are circumferentially spaced to define flow channels. Accordingly, in the normal centered position of the by-pass stopper, the chambers are sealed from on another and this is the position of the parts prior to use. It has been found that this arrangement provides an effective seal guaranteeing a long shelf life for the syringe and insuring stability of the components stored therein.

When it is desired to reconstitute the medicament, displacement of the discharge plunger toward the discharge end of the syringe increases the force of the diluent on the back face of the by-pass stopper, thereby displacing the by-pass stopper a predetermined small distance to a point where the internal rib separating the chambers engages the outer portion of the projections, thereby defining diluent channels or passageways between the projections permitting flow of diluent from the diluent chamber to the powder chamber. The by-pass stopper remains in this position because the frictional resistance between the syringe barrel rib and the projections is greater than the diluent fluid pressure on the by-pass stopper. This permits expelling all of the diluent from the diluent compartment until the discharge plunger abuts the rear face of the by-pass stopper. In this position all of the diluent has been transferred. The user then shakes the syringe to thoroughly mix the components of the medicament, aspirates the syringe to remove any air and now the reconstituted medicament is ready for delivery to the patient.

The syringe assembly of the present invention is of simplified construction which is easy and economical to manufacture since it is comprised of relatively few components. The single barrel, two compartment syringe system of the present invention insures hermetic sealing of the components thereby providing a long and stable shelf life. The specific details and arrangement of the assembly insure complete mixing of all of the components which is important in maintaining the efficacy of a given medicament.

Still another object of the present invention is to provide a single barrel two compartment syringe characterized by novel features of construction and arrangement wherein the barrel has virtually no surface protrusions and is therefore more adaptable for labeling as contrasted with the prior art external by-pass devices which make labeling much more difficult.

Still another object of the present invention is to provide a relatively simple design which is easy and economical to manufacture and assemble and is extremely effective in not only providing a seal between the chambers and guarantee a relatively long shelf life for two component medicaments, but also a design wherein the diluent can be completely evacuated from behind the by-pass stopper and fully mixed with the powder medicament before injection.

It is a further object of the present invention to provide a method for dispersing two components to be combined prior to discharge using a syringe. This object is achieved by the features according to claim 4.

### BRIEF DESCRIPTION OF THE DRAWINGS

These and other objects of the present invention and the various features and details of the operation and construction thereof are hereinafter more fully set forth with reference to the accompanying drawings, wherein;
Fig. 1 is a plan view of a two compartment syringe assembling in accordance with the present invention;
Fig. 2 is an enlarged, fragmentary transverse sectional elevational view taken on lines 2-2 of Fig. 1, showing material details of the two compartment syringe assembly prior to use;
Fig. 3 is an enlarged, isometric view of one embodiment of by-pass stopper of this invention;
Fig. 4 is an enlarged, side elevational view of the by-pass stopper.
Fig. 5 is a right hand side elevational view of the by-pass stopper shown in Fig. 4.
Fig. 6 is an enlarged, stepped sectional view taken on step line 6-6 of Fig. 2, showing details of the internal by-pass stopper engaging the sealing ring of the syringe barrel;
Figs. 7a-7e illustrate the sequential functions of the syringe assembly and associated components during a medicament reconstituting cycle;
Fig. 8 is a side, elevational view of a modified embodiment of the by-pass stopper of this invention;
Fig. 9 is a right hand, elevational view of the by-pass stopper shown in Fig. 8;
Fig. 10 is an enlarged, isometric view of the by-pass stopper shown in Fig. 8;
Fig. 11a is a plan view of the syringe of the present invention and the alternative embodiment of by-pass stopper;
Figs. 11b-11c are the sequential views of the components of a syringe embodying the modified embodiment by-pass stopper during a medicament reconstituting cycle.

### DETAILED DESCRIPTION OF THE PREFERRED EMBODIMENTS

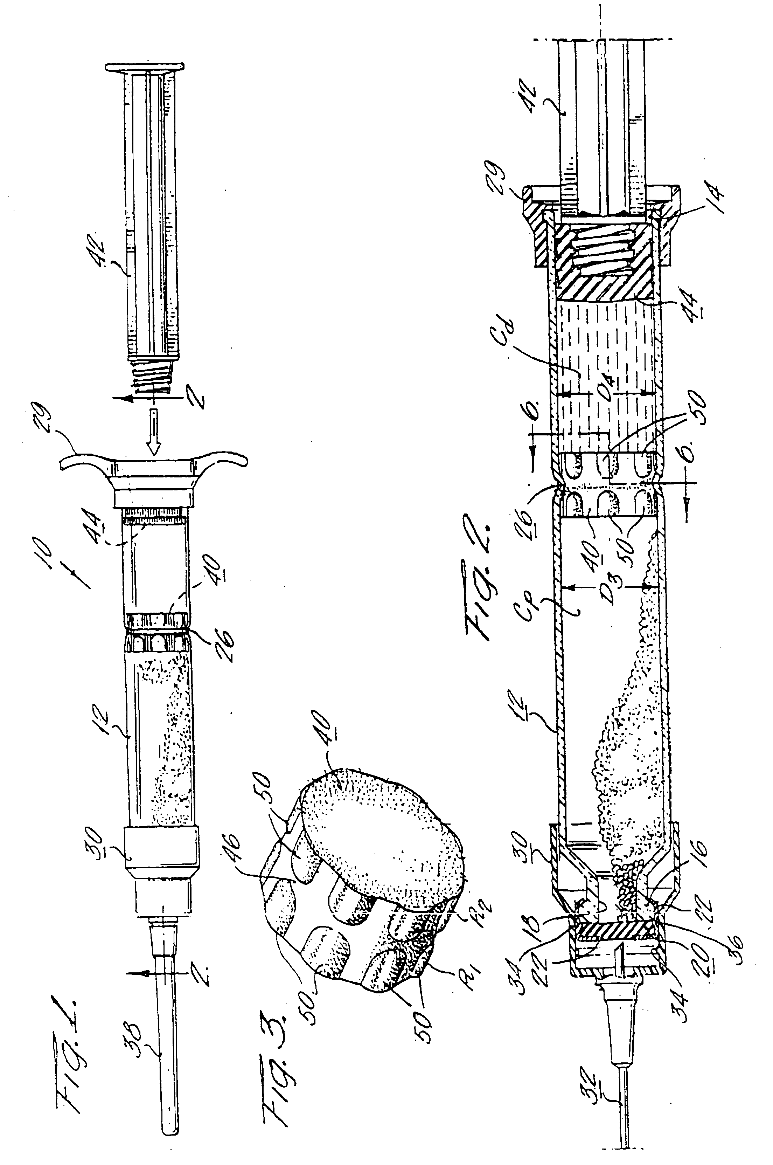

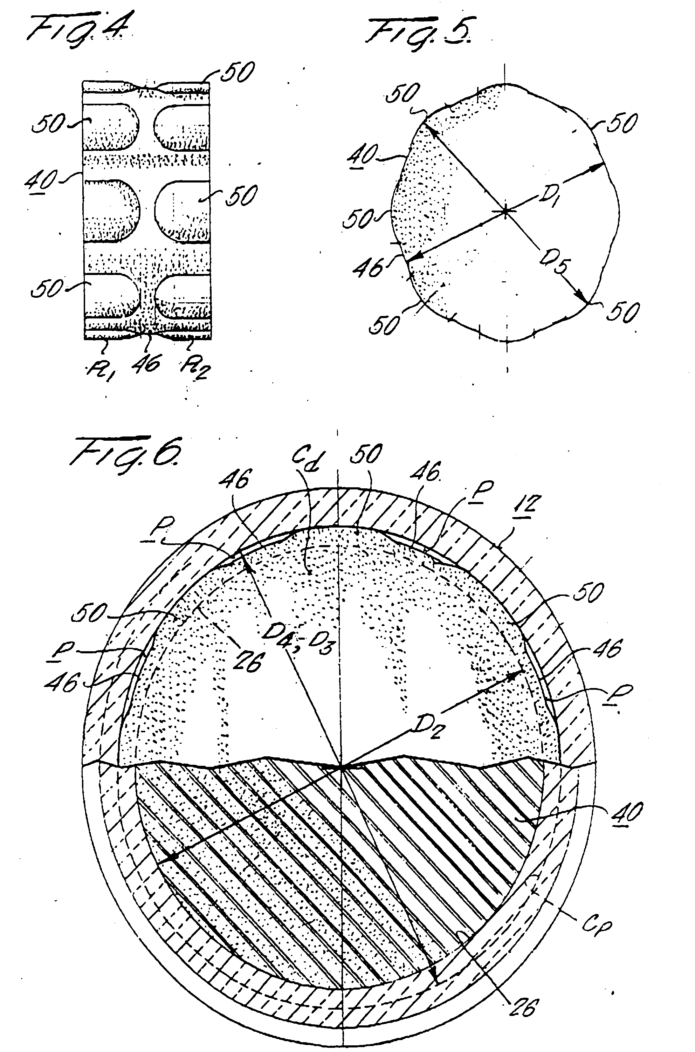

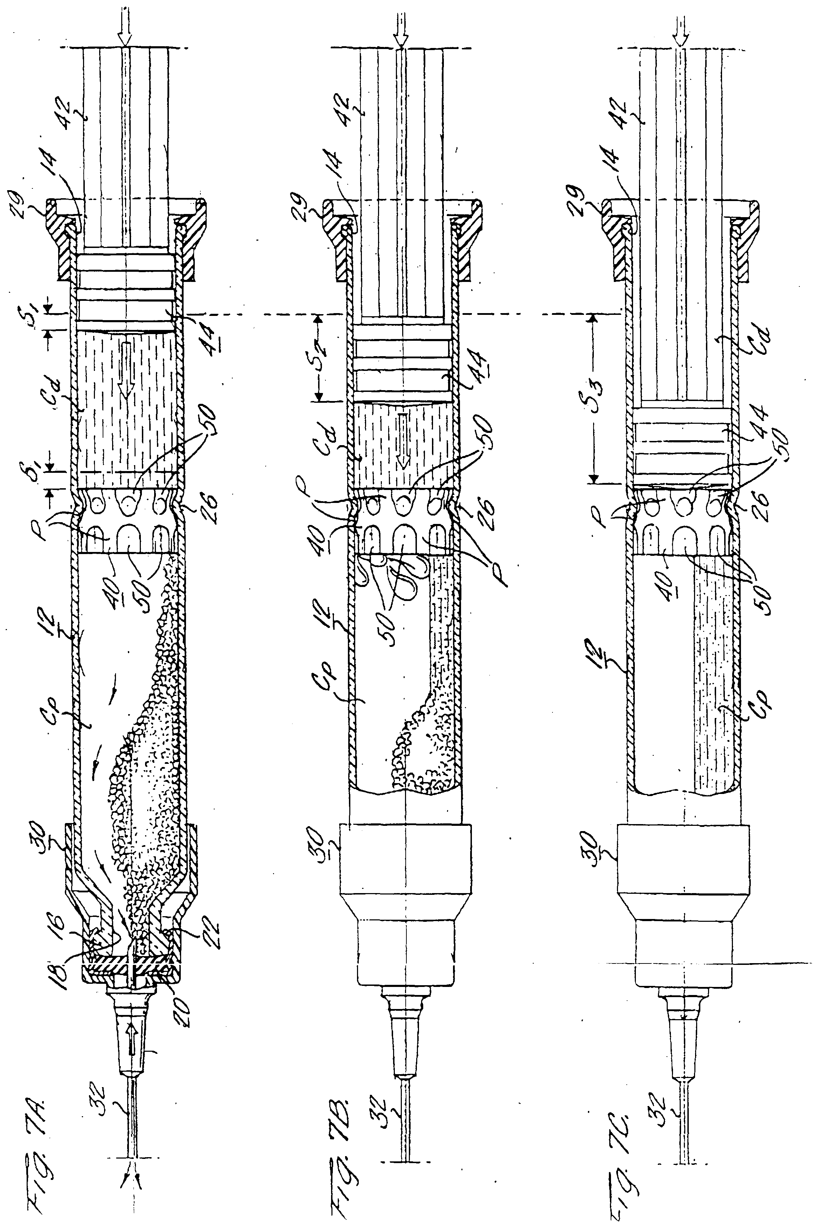

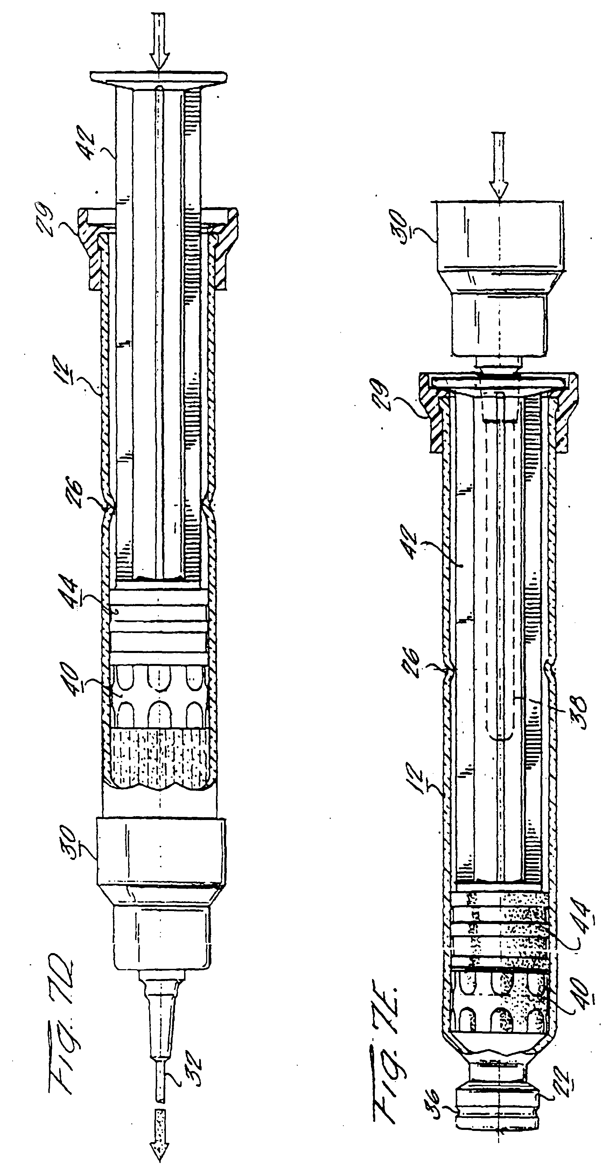

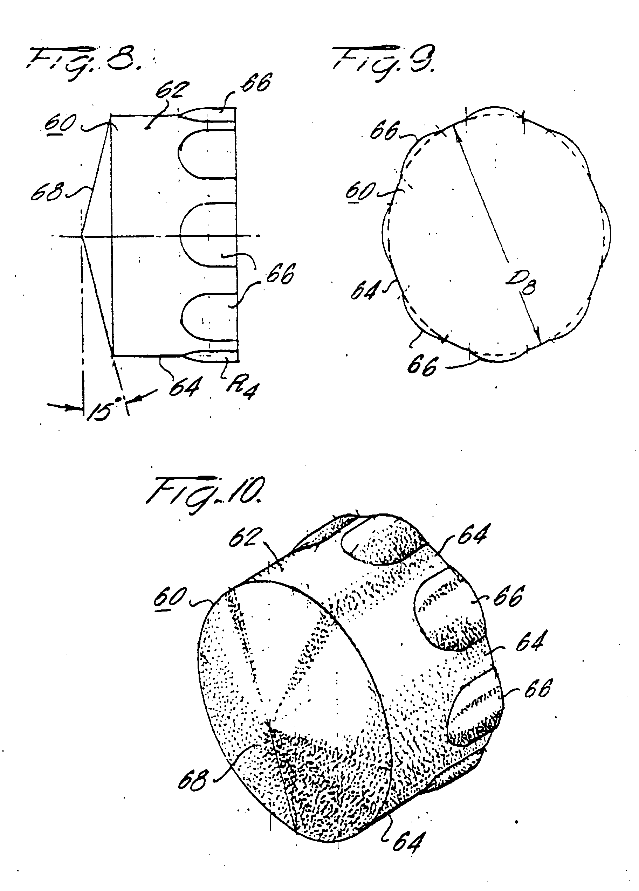

Referring now to the drawings and particularly to Figs. 1-7c, there is shown a syringe assembly in accordance with the present invention which is generally designated by the numeral **10**. The syringe assembly is a single barrel, two compartment syringe which as illustrated in Figs. 1 and 2 comprises an elongated hollow syringe band or body **12** usually made of glass open at its inner end as at **14** and having a reduced finish **16** at its forward end. The discharge opening **18** is normally sealed by a conventional circular disk-type seal **20** made of a flexible, puncturable material, such as rubber, supported in place over the discharge opening **18** by an aluminum seal **22** which is crimped over the finish to hold the seal **20** in place. The aluminum seal **22** has a central opening **22** in its end face to permit puncturing the disk seal **20** when it is desired to activate the syringe and dispense the contents. The assembly further includes a cup shaped needle hub **30** which mounts double ended needle **32**. The outer end **32**_{**a**} of the needle **32** is covered by a needle sheath **38** and is normally seated on the discharge end of the syringe barrel **12** in the position shown in Fig. 2 with the inner sharp end **32**_{**b**} of the needle **32** spaced from the disk seal **20**. The needle hub **30** has a pair of spaced circumferentially extending ribs **34** which engage in a groove **36** in the aluminum seal or cap **22** holding the needle hub **30** in an unarmed position, as shown in Fig. 2 or an armed position as shown in Fig. 7a.

The syringe barrel **12**, in the present instance, is divided into a powder medicament compartment **C**_{**p**} and a diluent compartment **C**_{**d**} by a by-pass stopper **40** frictionally held by means of a circumferentially extending inwardly directed bead **26** formed in the syringe body **12**. The by-pass stopper seals the compartments **C**_{**p**} and **C**_{**d**} from one another as explained in more detail hereafter. The stopper is made of an elastomeric material, such as a rubber compound conventionally used for forming plunger tips and seals in syringes, and, as will be understood by one of ordinary skill in the art, made from a formulation which the pharmaceutical manufacturer knows is compatible with the medicament and diluent.

The syringe assembly further includes a plunger rod **42** which threadedly mounts a discharge plunger **44** which engages in the inner end of the syringe barrel. A conventional finger grip **29** is mounted on the inner end of the syringe barrel and defines a stop or limit for the discharge plunger **44**.

Considering now briefly readying the syringe for use, the needle cup **30** is pushed inwardly causing the inner sharp end **32**_{**a**} of needle **32** to pierce stopper **20**, providing an air path between medicament chamber **C**_{**p**} and ambient environment. The plunger rod **42** may then be pressed inwardly to displace the discharge plunger **44** forwardly to create a displacement force on the rear face of the by-pass stopper **40** to displace it slightly to the position shown in Fig. A and create flow channels permitting flow of diluent into the powder medicament chamber **C**_{**p**}. After all of the diluent has been evacuated, the contents are mixed in the forward chamber **C**_{**p**} and the medicament product is ready for injection. The contents can be dispensed by forward movement of the discharge plunger in the manner shown in Figs. 7d and 7e until the contents have been fully charged. When the medicament has been fully discharged the plunger rod **42** terminal end sets in the finger grip **30** the needle cup **30** may then be removed from the syringe body and inserted in the plunger rod **42**, providing a safe throw away package.

Considering now more specifically the by-pass stopper **40**, the stopper as best shown in Figs. 3 and Figs. 4-6 inclusive comprises a series of axially extending, circumferentially spaced projections **50** which in the present instance are arranged in two axially spaced rows **R**_{**1**} and **R**_{**2**} to define a generally cylindrical sealing surface **46** between the rows **R**_{**1**} and **R**_{**2**} of projections. The sealing surface **46** is preferably of a diameter **D**_{**1**} greater then the diameter **D**_{**2**} of the radially inwardly circumferentially extending bead **26** defining the separation point between the powder and diluent chambers. The diameter **D**_{**1**} of the sealing surface **46** is also preferably less then the diameters **D**_{**3**} and **D**_{**4**} of the powder and diluent chambers **C**_{**p**} and **C**_{**d**} which in the preferred form are of the same diameter. A circular trace through the apex of the ribs is of a diameter **D**_{**5**} greater then the diameter of the **D**_{**2**} of circumferential bead **26** so that when the by-pass stopper **40** is displaced to the position shown in Fig. 7a during the initial activation of the discharge plunger, flow passageways **P** form between the ribs **50** as shown in Fig. 7a to permit diluent to flow from the diluent compartment **C**_{**p**} to the powder compartment **C**_{**p**}. Further, the diametral relationships and materials of the by-pass stopper **40** and barrel **12** are such that the frictional force holding the by-pass stopper in the position in Fig. 7a is greater then the hydraulic force on the back face of the by-pass stopper **40** during the displacement stroke of the discharge plunger **44**. In this manner, all of the diluent is evacuated to the powder chamber **C**_{**p**} with the by-pass stopper in a static position until the discharge plunger **44** abuts the rear face of the by-pass stopper **40** in the manner shown in Fig. 7c. The by-pass stopper **40** is then pushed beyond the circumferential bead **26** during the medicament discharged stroke and rides in front of the discharge plunger **44** in the manner shown in Fig. 7d and 7e.

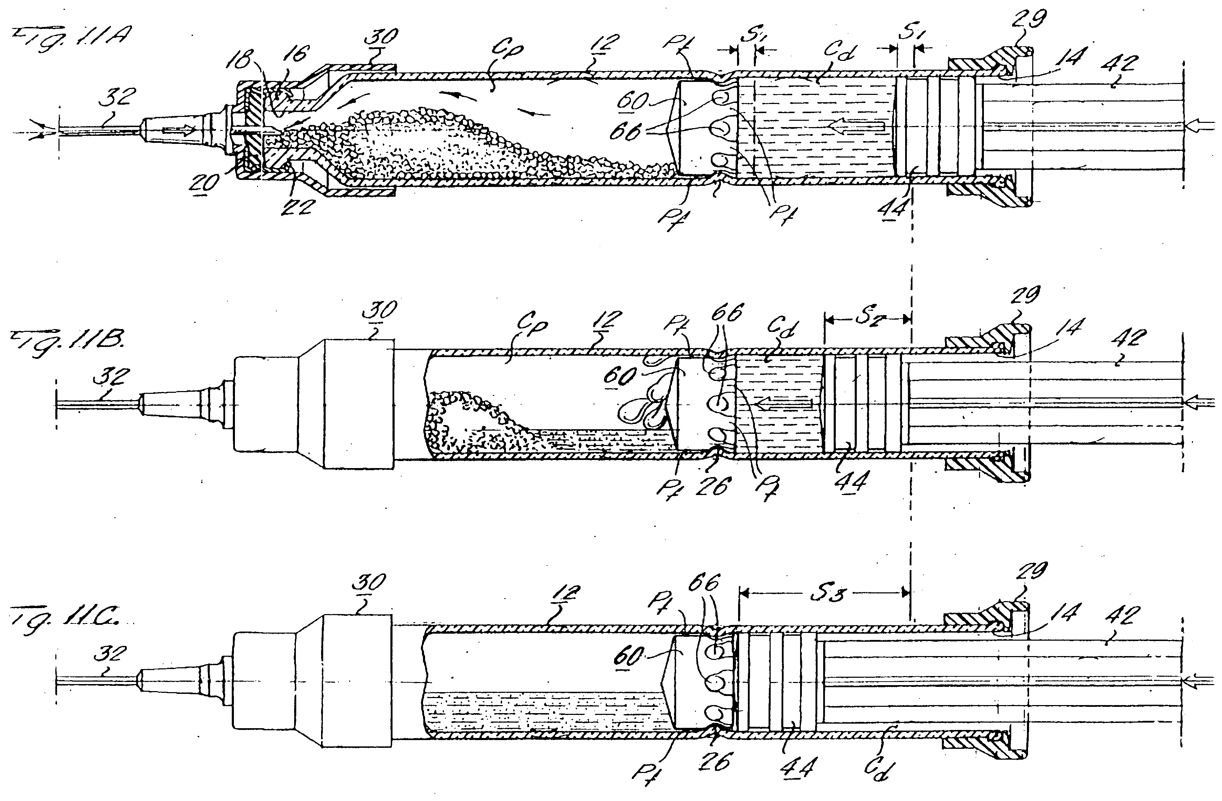

There is shown in Figs. 8-10 inclusive another embodiment of by-pass stopper **60** in accordance with the present invention. This by-pass stopper **60** functions in the same manner to the by-pass stopper previously described in that it provides a seal between the powder chamber and diluent chamber **C**_{**p**} and **C**_{**d**} and upon displacement creates flow channels for mixing the diluent and powder medicament in the forward chamber. In the present instance, the by-pass stopper has a generally cylindrical forward or nose portion **62**, the outer periphery of which defines a sealing surface **64** having a diameter **D**_{**8**} slightly greater then the diameter **D**_{**2**} of the circumferentially extending inwardly directed bead **26**. A single row **R**_{**4**} of circumferentially spaced, axially extending ribs **66** are provided on the outer peripheral surface which are of generally arcuate shape in cross section as shown in Fig. 9 and define therebetween a series of axial flow passages **P**_{**f**}. The sealing surface **64** is preferably of a diameter slightly less than the diameter of the powder medicament and the diluent chambers **C**_{**p**} and **C**_{**d**} to facilitate flow between the chambers when the by-pass stopper **60** is initially displaced and also to facilitate retention of the by-pass stopper **60** in the partially displaced position shown in Figs. 11a, 11b and 11c. In the present instance, the front face **68** of the by-pass stopper **60** is conically shaped and forms an angle of about 15° to the axis of the plunger. The by-pass stopper **60** is particularly suited for very fine powder medicament products which tend to produce a frosted look to the stopper when the double row by-pass stopper **40** is used with fine powder medicaments. The fine powder tends to infiltrate between the projections **50** in the spaces **P** formed between the by-pass stopper sealing surface **46** and the inner wall of the chamber **C**_{**p**} creating an undesirable frosted look, which the user may attribute to diluent leakage. The modified by-pass stopper **60** tends to keep powder or lyophilization dust out of the region between the stopper and the barrel.

Even though particular embodiments of the present invention have been illustrated and described herein, it is not intended to limit the invention and changes and modifications may be made therein with the scope of the following claims.

## Claims

1. A syringe assembly comprising:
An elongated hollow tubular barrel (12) having a discharge opening (18) at a distal end; and
means defining a radially inwardly directed circumferentially extending rib (26) in the inner peripheral surface of said barrel (12) dividing said barrel (12) into a first medicament chamber (Cₚ) on one side of said rib (26) and a second diluent chamber (C_{d}) on the opposite side of said rib (26);
a by-pass stopper (40; 60) engageable interiorly of said barrel (12) having a circumferentially extending exterior sealing surface (46; 64) of a predetermined diameter (D₁; D₈) greater than the diameter (D₂) of the rib (26) to provide a seal between said chambers (Cₚ, C_{d}) when engaged with the rib (26);
characterized in that
the by-pass stopper (40; 60) comprises axially extending, circumferentially spaced projections (50; 66) defining at least one axially extending flow path on at least the proximal side of said sealing surface (46) providing fluid communication between said chambers (Cₚ, C_{d}) when said stopper is displaced axially relative to the rib (26).

2. The syringe assembly of Claim 1, wherein said by-pass stopper (40) includes projections (50) circumferentially spaced on both sides of a central portion, said central portion circumferentially engaging said sealing rib (26).

3. The syringe assembly of Claim 1, wherein said by-pass stopper (60) includes a plurality of projections (66) circumferentially spaced thereon only on the proximal side of the by-pass stopper (60).

4. A method for mixing two components to be combined prior to discharge using a syringe, comprising the steps of:
providing a syringe (10) including a liquid in a proximal chamber (C_{d}) and a medicament mixable with said liquid in a distal chamber (Cₚ) and including a body having a radially inwardly directed internal sealing rib (26) to define said distal and proximal chambers (Cₚ, C_{d}) and a by-pass stopper (40; 60) having a portion (46; 64) sealingly separating said chambers, and a plurality of axially extending, circumferentially spaced projections on at least one said of said sealing portion (46; 64) defining by-pass channels;
actuating a plunger rod (42) for moving said by-pass stopper (40; 60) axially to displace the sealing portion (46; 64) from the rib (26) to permit flow of liquid through said by-pass channels from said proximal chamber (C_{d}) to said distal chamber (Cₚ) for mixing said medicament and said liquid.

5. The method of Claim 4 wherein said by-pass (40) stopper includes projections (50) circumferentially spaced on both sides of a cencral portion, said central portion circumferentially engaging said sealing rib (26) to provide a sealing engagement therebetween.

6. The method of Claim 4 wherein said by-pass stopper (60) includes a plurality of projections (66) circumferentially spaced thereon only on the proximal side of the by-pass stopper (40).

7. The method of Claim 4 wherein said internal by-pass stopper (40) includes a cone shaped distal end (68) facing said distal chamber (Cₚ).

8. The method of Claim 4 which further includes a needle hub assembly (30) for mounting a needle (32) on said device.

## Patentansprüche

1. Spritzenanordnung, die aufweist:
einen länglichen hohlen zylinderförmigen Behälter (12) mit einer Ausstoßöffnung (18) an einem distalen Ende; und
Mittel, die eine radial nach innen gerichtete, sich entlang des Umfangs erstreckende Rippe (26) an der inneren Oberfläche des Kolbens (12) definieren, die den Behälter (12) in eine erste Medikamentenkammer (Cₚ) an einer Seite der Rippe (26) und eine zweite Verdünnungsmittel-Kammer (C_{d}) an der entgegengesetzten Seite der Rippe (26) unterteilt;
einen Bypass-Stopfen (40; 60), der im Inneren des Behälters (12) angreifen kann und der eine sich entlang des Umfangs erstreckende äußere Dichtfläche (46, 64) eines vorgegebenen Durchmessers (D₁; D₈), der größer ist als der Durchmesser (D₂) der Rippe (26), aufweist, um eine Dichtung zwischen den Kammern (Cₚ, C_{d}) zu bilden, wenn er an der Rippe (26) anliegt;
**dadurch gekennzeichnet,** dass der Bypass-Stopfen (40; 60) sich axial erstreckende, entlang des Umfangs beabstandet angeordnete Vorsprünge (50; 66) aufweist, die wenigstens einen sich axial erstreckenden Durchflusspfad wenigstens an der proximalen Seite der Dichtfläche (46) definieren, um eine Flüssigkeitsverbindung zwischen den Kammern (Cₚ, C_{d}) zur Verfügung zu stellen, wenn der Stopfen axial relativ zu der Rippe (26) verschoben wird.

2. Spritzenanordnung nach Anspruch 1,
bei der der Bypass-Stopfen (40) Vorsprünge (50) aufweist, die beabstandet entlang des Umfangs an beiden Seiten eines zentralen Abschnitts angeordnet sind, wobei der zentrale Abschnitt entlang des Umfangs an der Dichtrippe (26) anliegt.

3. Spritzenanordnung nach Anspruch 1,
bei der der Bypass-Stopfen (60) eine Vielzahl von Vorsprüngen (66) aufweist, die beabstandet an dessen Umfang nur an der proximalen Seite des Bypass-Stopfens (60) angeordnet sind.

4. Verfahren zum Mischen zweier Komponenten, die vor dem Ausstoßen zusammengebracht werden müssen, unter Verwendung einer Spritze, wobei das Verfahren die Schritte aufweist:
Bereitstellen einer Spritze (10) mit einer Flüssigkeit in einer proximalen Kammer (C_{d}) und einem mit der Flüssigkeit mischbaren Medikament in einer distalen Kammer (Cₚ) und mit einem Körper, der eine radial nach innen gerichtete innere Dichtrippe (26) aufweist, um die distale und proximale Kammer (Cₚ, C_{d}) zu definieren, und mit einem Bypass-Stopfen (40; 60), der einen Abschnitt (46, 64), der die Kammern dichtend trennt, und eine Vielzahl von sich axial erstreckenden, entlang des Umfangs beabstandet angeordneten Vorsprüngen an wenigstens einer Seite des Dichtabschnitts (46; 64), welche Bypass-Kanäle definieren, aufweist;
Betätigen einer Kolbenstange (42) zum Verschieben des Bypass-Stopfens (40; 60) in axialer Richtung, um den Dichtungsabschnitt (46; 64) weg von der Rippe (26) zu verschieben, um einen Flüssigkeitsdurchfluss durch die Bypass-Kanäle von der proximalen Kammer (C_{d}) zu der distalen Kammer (Cₚ) zu ermöglichen, um das Medikament und die Flüssigkeit zu mischen.

5. Verfahren nach Anspruch 4,
bei dem der Bypass-Stopfen (40) Vorsprünge (50) aufweist, die beabstandet entlang des Umfangs an beiden Seiten eines zentralen Abschnitts angeordnet sind, wobei der zentrale Abschnitt in Umfangsrichtung an der Dichtrippe (26) anliegt, um dazwischen eine Dichtvorrichtung zu bilden.

6. Verfahren nach Anspruch 4,
bei dem der Bypass-Stopfen (60) eine Vielzahl von Vorsprüngen (66) aufweist, die daran entlang des Umfangs beabstandet nur an der proximalen Seite des Bypass-Stopfens angeordnet sind.

7. Verfahren nach Anspruch 4,
bei dem der interne Bypass-Stopfen (40) ein konisch geformtes distales Ende (68) aufweist, das in Richtung der distalen Kammer (Cₚ) zeigt.

8. Verfahren nach Anspruch 4,
welches weiterhin eine Nadelbefestigungsvorrichtung (30) zum Befestigen einer Nadel (32) an der Vorrichtung aufweist.

## Revendications

1. Dispositif de seringue comprenant :
- un cylindre tubulaire creux allongé (12) comportant une ouverture de décharge (18) à une extrémité distale;
- des moyens définissant une nervure s'étendant circonférentiellement (26) dirigée radialement vers l'intérieur dans la surface périphérique intérieure du cylindre (12) pour diviser ce cylindre (12) en une première chambre de médicament (Cₚ) du côté de la nervure (26), et une seconde chambre de diluant (C_{d}) du côté opposé de la nervure (26) ; et
- un obturateur de dérivation (40 ; 60) pouvant s'engager à l'intérieur du cylindre (12) et comportant une surface d'étanchéité extérieure s'étendant circonférentiellement (46 ; 64) présentant un diamètre prédéterminé (D₁ ; D₈) supérieur au diamètre (D₂) de la nervure (26), de manière à former un joint d'étanchéité entre les chambres (Cₚ, C_{d}) lorsqu'il est engagé sur la nervure (26).
caractérisé en ce que
l'obturateur de dérivation (40 ; 60) comprend des saillies (50 ; 66) circonférentiellement espacées s'étendant axialement qui définissent au moins un chemin d'écoulement s'étendant axialement au moins du côté distal de la surface d'étanchéité (46), en créant une communication de fluide entre les chambres (Cₚ, C_{d}) lorsque l'obturateur est déplacé axialement par rapport à la nervure (26).

2. Dispositif de seringue selon la revendication 1,
dans lequel
l'obturateur de dérivation (40) comprend des saillies (50) espacées circonférentiellement des deux côtés d'une partie centrale, cette partie centrale s'engageant circonférentiellement sur la nervure d'étanchéité (26).

3. Dispositif de seringue selon la revendication 1,
dans lequel
l'obturateur de dérivation (60) comprend un certain nombre de saillies (66) circonférentiellement espacées sur celui-ci uniquement du côté proximal de cet obturateur de dérivation (60).

4. Procédé de distribution de deux composants devant être combinés avant d'être déchargés en utilisant une seringue, comprenant les étapes consistant à :
- utiliser une seringue (10) contenant un liquide dans une chambre distale (C_{d}) et un médicament mélangeable au liquide dans une chambre proximale (Cₚ) cette seringue comprenant un corps muni d'une nervure d'étanchéité interne dirigée radialement vers l'intérieur (26) pour définir la chambre proximale (Cₚ) et la chambre distale (C_{d}), ainsi qu'un obturateur de dérivation (40 ; 60) comportant une partie (46 ; 64) séparant les chambres de manière étanche, et un certain nombre de saillies circonférentiellement espacées, s'étendant axialement, au moins sur la partie d'étanchéité (46 ; 64) définissant des canaux de dérivation ;
- actionner une tige de plongeur (42) pour déplacer l'obturateur de dérivation (40 ; 60) axialement de manière à écarter la partie d'étanchéité (46 ; 64) de la nervure (26) afin de permettre l'écoulement de liquide à travers les canaux de dérivation de la chambre distale (C_{d}) vers la chambre proximale (Cₚ), de manière à permettre le mélange du médicament et du liquide ; et
- distribuer le médicament et le liquide mélangés.

5. Procédé selon la revendication 4,
dans lequel
l'obturateur de dérivation (40) comprend des saillies (50) circonférentiellement espacées des deux côtés d'une partie centrale, cette partie centrale s'engageant cironférentiellement sur la nervure d'étanchéité (26) pour former un engagement d'étanchéité entre les deux.

6. Procédé selon la revendication 4,
dans lequel
l'obturateur de dérivation (60) comprend un certain nombre de saillies (66) circonférentiellement espacées sur celui-ci, uniquement du côté proximal de cet obturateur de dérivation (60).

7. Procédé selon la revendication 4,
dans lequel
l'obturateur de dérivation interne (60) comprend une extrémité proximale en forme de cône (68) venant en face de la chambre proximale (Cₚ).

8. Procédé selon la revendication 4,
comprenant en outre
un dispositif de moyeu à aiguille (30) pour monter une aiguille (32) sur ce dispositif.
